# EUROPEAN PATENT SPECIFICATION

(11) **EP 0 606 366 B1**
(45) Date of publication and mention of the grant of the patent: **04.06.2003**
(21) Application number: 92921255.3
(22) Date of filing: 28.09.1992
(51) Int. Cl.: C07D 487/04, C07D 491/048, A61K 31/40

(54) **SUBSTITUTED PHENSERINES AND PHENYLCARBAMATES OF (-)-ESEROLINE, (-)-N1-NORESEROLINE, AND (-)-N1-BENZYLNORESEROLINE; AS SPECIFIC INHIBITORS OF ACETYLCHOLINESTERASE**
SUBSTITUIERTE PHENSERINE UND PHENYLCARBAMATE VON (-)-ESEROLIN, (-)-N1-NORESEROLIN UND (-)-N1-BENZYLNORESOROLIN ALS SPEZIFISCHE ACETYLCHOLINESTERASE-INHIBITOREN
PHENSERINES ET PHENYLCARBAMATES SUBSTITUES DE (-)-ESEROLINE, (-)-N1-NORESEROLINE, ET (-)-N1-BENZYLNORESEROLINE UTILISES COMME INHIBITEURS SPECIFIQUES DE L'ACETYLCHOLINESTERASE

(30) Priority: 26.09.1991 US 765746; 31.03.1992 US 861329
(43) Date of publication of application: 20.07.1994
(73) Proprietor: THE GOVERNMENT OF THE UNITED STATES OF AMERICA as represented by THE SECRETARY of the DEPARTMENT OF HEALTH AND HUMAN SERVICES, Washington, DC 20201 (US)
(72) Inventor: BROSSI, Arnold, Bethesda, MD 20817 (US); BRZOSTOWSKA, Malgarzota, 60-780 Poznan (PL); RAPOPORT, S., Washington, DC 20016 (US); GREIG, Nigel, Silver Spring, MD 20906 (US); HE, Xiao-shu, Branford, CT 06405 (US)
(74) Representative: Rackham, Anthony Charles
(86) International application number: PCT/US92/08228
(87) International publication number: WO 93/006105

(56) References cited:
- EP-A- 0 154 864
- EP-A- 0 253 372
- HELVETICA CHIMICA ACTA vol. 74, no. 4, 19 June 1991, BASEL CH pages 761 - 766 Q.-S. YU ET AL. 'Physovenines: Efficient synthesis of (-)- and (+)-physovenine and synthesis of carbamate analogues of (-)-physovenine. Anticholinesterase activity and analgesic properties of optically active physovenines'

## Description

The present invention relates to improvements in the treatment of diseases, and more particularly to compounds which exhibit selective inhibition of acetylcholinesterase.

Physostigmine, also called eserine, and particular derivatives of physostigmine are anti-cholinesterase inhibitors which are well known. Such well known compounds are also useful in the treatment of glaucoma, Myasthenia Gravis, Alzheimer's disease and as antidotes against poisoning with organophosphates.

Physostigmine was introduced into England in 1840 by Daniell (a British medical officer) in the form of the Calabar bean. The compound itself was first isolated by Jobst and Hesse in 1864. Physostigmine has been used as a treatment for glaucoma, and to reverse atropine-induced coma during the last century. Recent uses for this compound and its derivatives have been as effective antidotes to several drugs which possess central anti-cholinergic properties.

During the last two decades, studies related to the acetylcholine-receptor-ion-channel complex of the neuromuscular junction have provided significant increases in knowledge of the receptor function. This membrane receptor has been readily available for study since nicotinic AChRs occur at very high densities in Torpedo and Electrophorus electric organs. Further, the understanding of the morphology and function of this receptor has been increased significantly by specific chemical probes for the different active sites of the receptor.

Nearly 20 years ago a significant discovery was made which helped in the study of this AChR. Alpha-bungarotoxin (Alpha-PGT) was obtained from snake venoms which binds irreversibly and specifically to the acetylcholine (ACh) recognition site on the nicotinic AChR. Alpha-PGT was such a highly selective probe that researchers were able to isolate and purify the different sub-units which comprise the nicotinic AChR. The sub-units were functionally reconstituted into artificial lipid membranes and were ultimately cloned.

Further sites on the nicotinic AChR were soon made available by the discovery of another class of toxins. These toxins were called histrionicotoxins and were isolated from the skin secretion of frogs in the family Dendrobatidae. The new sites available because of the histrionicotoxins were discovered to be responsible for the allosteric alterations or non-competitive blockage of neuromuscular transmission. These sites are distinct from the acetylcholine recognition site discovered through the alpha-PGT probe and are thought to be located on the ion channel component of the AChR.

Further, other drugs demonstrate the ability to modify non-competitively the activation of the AChR. Examples of such drugs are distinct and well known pharmacological agents which act on the peripheral nervous system as well as in the central nervous system. In particular, tricyclic anti-depressants, phenothiazine antipsychotics, the hallucinogenic agent Phencyclidine (PCP), local anesthetics, antimuscarinics, anticholinesterase agents and similar compounds to mention but a few.

Further ways for studying AChR are available due to microscopic kinetic models and biochemical rapid mixing methods to study permeability changes initiated by the binding of agonist molecules and conformational transitions of nicotinic receptor molecules.

The agonist recognition site at the nicotinic ACh receptor lias been reported as having strong stereospecificity. This conclusion is based on the study of optical isomers of certain semi-rigid agonists, see for example Spivak et al., Mol. Pharmacol., Vol. 23, pages 337-343 (1983).

Conversely, the ion channel sites are apparently not stereospecific. This conclusion is based on the similar quantitative and qualitative actions of enantiomers of perhydrohistrionicotoxin at the nicotinic AChR, see for example Spivak et al, FEBS Lett. Vol. 163, pages 189-193 (1983).

It has been discovered that the natural isomer of physostigmine has blocking properties as well as agonist properties at the neuromuscular AChR. By contrast (+)-physostigmine shows only negligible inhibition of cholinesterase (ChE). See Brossi et al., FEBS Lett., Vol. 201, pages 190-192 (1986).

Even though (+)-physostigmine has only negligible ChE inhibitory activity it is very effective as a protective pretreatment drug against multiple lethal doses of sarin, see Albuquerque et al, Fundam. Appl. Caltoxicol., Vol. 5, pages 182-203 (1985). The observed beneficial protection appears to be due to direct interactions of the carbamates with the postsynaptic nicotinic AChR. The protective effectiveness of the carbamates against organophosphates appears to be related to the direct ability of the carbamates to decrease the hyperactivation caused by accumulation of the neurotransmitter.

Helv.Chim.Act. 74 (1991), 761-766 discloses (-)-5-O-(phenylcarbamoyl)physovenol and (-)-5-O- (cumylcarbamoyl)-physovenol. The former of these compounds is shown to be much more active against AChE than BChE whereas the latter compound is shown to be far more active against BChE than AChE.

EP-A-0154864 discloses various physostigmine derivatives which contain an alkylcarbamoyl moiety. These compounds have been shown to have AChE inhibition properties.

EP-A-0253372 discloses, inter alia, 3'-chlorophenylcarbamoyleseroline, 4'-chlorophenylcarbamoyleseroline, 2',6'-dimethylphenylcarbamoyleseroline and 4'-nitrophenyl carbamoyleseroline. These compounds are said to inhibit cholinesterase and data is provided which shows that the first of these compounds selectively inhibits brain AChE as opposed to serum AChE. However, no phenylcarbamoyleseroline or phenylcarbamoyl-Nl-noreseroline derivatives are disclosed in which the phenyl ring of the phenylcarbamoyl moiety is unsubstituted, substituted at the 2-position only or substituted at the 2- and 4-positions. Moreover, there is nothing in EP-A-0253372 which teaches or suggests that such compounds would exhibit selective inhibition of AChE over BChe.

The above information, available due to the research in this field, is important in the evaluation of potential new pharmacological agents for treating cholinergic disorders, for example, Myasthenia Gravis and Alzheimer's disease. Potential agents can be evaluated for potency in vitro by testing the agents against electric eel acetylcholinesterase (AChE) and human plasma butyrylcholinesterase (BChE).

Of the two enzymes known to hydrolyze acetylcholine (ACh) in vivo, AChE, which is found in red blood cells, in the brain and in nerve tissues, seems to be more specific than BChE which is found in serum, pancreas and in the liver. It, however, has not previously been shown in the art that compounds which selectively inhibit one of the two enzymes more than the other would offer a medical advantage. The natural alkaloid (-)-physostigmine, its potential metabolite (-)-(N1)-norphysostigmine, and the natural alkaloid physovenine which are used as biological standards in this art area inhibit AChE and BChE in vitro similarly at similar concentrations.

Accordingly, there is need in the art for highly selective agents active against one of AChE and BChE and not very potent against the other which may lead to better treatment of a particular cholinergic disorder and minimize negative side effects. Such compounds would be of great medical importance in the treatment of cholinergic disorders.

### Summary of the Invention

It is an object of the present invention to overcome the difficulties in the prior art as set forth in the background of the invention.

It is another object of the present invention to provide highly potent and selective cholinergic agonist and blocking compounds.

It is a further object of the present invention to provide improvements in therapy relative to cholinergic diseases such as glaucoma, Myasthenia Gravis, Alzheimer's disease, and organophosphate poisoning.

It is a still further object of the present invention to provide compounds with selective acetylcholinesterase activity.

It is a yet further object of the present invention to provide compounds having the following formula: 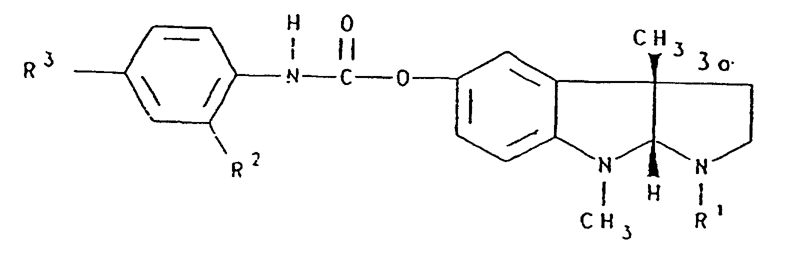 wherein R¹ is H or a -CH₃ group,
R² is a straight or branched chained C₁-C₃ alkyl,
R³ is H or a straight or branched C₁-C₃ alkyl,
including isomeric forms and pharmaceutically acceptable salts.

### Brief Description of the Figure

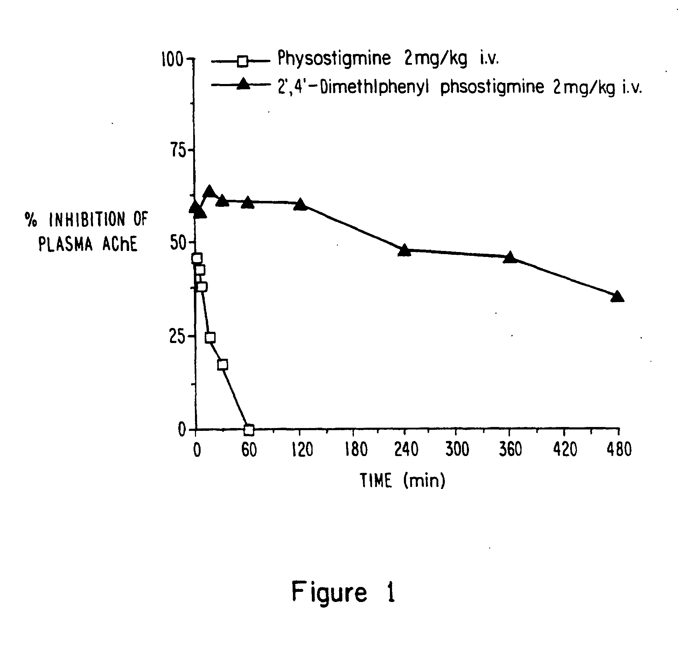

Figure 1 illustrates the time-dependent inhibition of plasma AChE in a rat host by physostigmine and its 2' ,4'-dimethylphenyl carbamate.

### Description of Preferred Embodiments

In accordance with this invention there are disclosed compounds of the formula 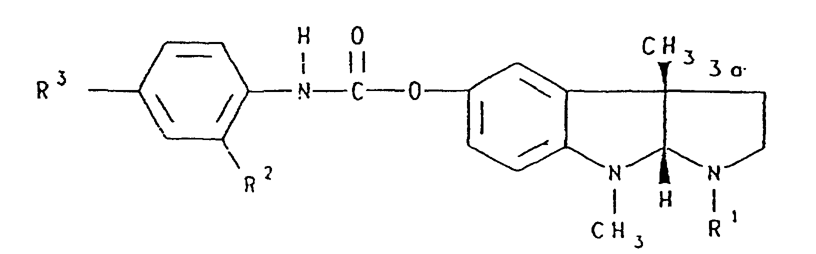 wherein R¹ is H or a -CH₃ group,
R² is a straight or branched chained C₁-C₃ alkyl,
R³ is H or a straight or branched C₁-C₃ alkyl, and pharmaceutically acceptable salts.

In one preferred group of compounds, R¹ is hydrogen whereas, in another preferred group of compounds, R¹ is -CH₃.

Even more preferred are compounds wherein R³ is hydrogen and R² is C₁₋₃-alkyl. Yet more preferred is where these two groups are independently -CH₃, CH₂-CH₃, -CH₂-CH₂-CH₃ and -CH(-CH₃)₂.

Preferably, the compound is selected from
(-)-2'-methylphenylcarbamoyleseroline;
(-)-2', 4'-dimethylphenylcarbamoyleseroline;
(-)-2'-ethylphenylcarbamoyleseroline;
(-)-2'-isopropylphenylcarbamoyleseroline;
(-)-2'-methylphenylcarbamoyl-N1-noreseroline;
(-)-2', 4'-dimethylphenylcarbamoyl-N1-noreseroline;
(-)-phenylcarbamoyl-N1-noreseroline;
and pharmaceutically acceptable salts thereof.

The above compounds are eseroline and (1) N-noreseroline carbamates having high potency in the inhibition of acetylcholinesterase and not butyrylcholinesterase. Thus, these carbamates were more specific for AChE.

Also preferred are compounds according to the present inverition in isomeric forms and pharmaceutically acceptable salts thereof. pharmaceutically acceptable salts can be, for example, the alkali metal, alkali earth and ammonium salt. Further, pharmaceutically acceptable organic and inorganic acid addition salts may be used. Other examples of acceptable salts are tartrates, fumarates, phosphates, salicyclates, and the like.

The compounds according to Formula I have asymmetric carbon atoms and can exist as optical isomers. For the purpose of this invention, the racemic mixtures and dextro and laevo forms are included within the present invention. Hence, the particular dextro and laevo rotatory form or a particular isomer is sometimes indicated as a preferred optical isomer in particular formulae according to the invention.

Other cholinesterase inhibitors are known in the prior art. Physostigmine and physovenine are optically active alkaloids with a (3aS)-absolute configuration at the chiral carbon atom C(3a). Both of these compounds are potent inhibitors of cholinesterases in vitro and in vivo, blocking the conversion of acetylcholine into choline reversibly. Physostigmine has been found to have useful medical applications in disorders which result in a malfunction of this process.

Surprisingly, the carbamates according to the present invention have shown high potency. Thus, phenylcarbamate derivatives according to the present invention are longer lasting and appear to be less toxic than other carbamate analogues in this art. Accordingly, the present compounds represent a significant advancement in the prior art.

Further, the above compounds according to the invention are useful as highly potent and selective cholinergic agonist and blocking pharmaceutical agents. Hence, the compounds of the present invention are useful in pharmaceutical compositions for systemic administration to human and animals in unit dosage forms, such as tablets, capsules, pills, powders, granules, suppositories, sterile parenteral solutions or suspensions, sterile non-parenteral solutions or suspensions, oral solutions or suspensions, oil and water or water in oil emulsions and the like, containing suitable quantities of the active ingredient. Topical application can be in the form of ointments, creams, lotions, jellies, sprays, douches, and the like. For oral administration either solid or fluid unit dosage forms can be prepared with the compounds of Formula I.

Compositions within the scope of the invention include compositions wherein the active ingredient is contained in an effective amount to achieve its intended purpose. The compounds can be administered in any pharmaceutically acceptable amount, for example, in amounts ranging from 0.001 gram to about 1 gram per kilogram of body weight. Based on the information which is presented herein, determination of effective amounts is well within the skill of the ordinary practitioner in the art. The compounds are generally useful in pharmaceutical compositions (wt%) of the active ingredient with a carrier or vehicle in the composition in about 0.1 to 99 wt% and preferably about 25-85 wt%.

Either fluid or solid unit dosage forms can be readily prepared for oral administration. For example, the compounds of Formula I can be admixed with conventional ingredients such as dicalcium phosphate, magnesium aluminum silicate, magnesium stearate, calcium sulfate, starch, talc, lactose, acacia, methyl cellulose and functionally similar materials as pharmaceutical excipients or carriers. The compounds according to the invention can also be administered as water soluble salts such as salicylates, oxalates, and such like. A sustained release formulation may optionally be used. Capsules may be formulated by mixing the compound with a pharmaceutical diluent which is inert and inserting this mixture into a hard gelatin capsule having the appropriate size. If soft capsules are desired a slurry of the compound with an acceptable vegetable, light petroleum or other inert oil can be encapsulated by making into a gelatin capsule.

Suspensions, syrups and elixirs may be used for oral administration of fluid unit dosage forms. A fluid preparation including oil may be used for oil soluble forms. A vegetable oil such as corn oil, peanut oil or safflower oil, for example, together with flavoring agents, sweeteners and any preservatives produces an acceptable fluid preparation. A surfactant may be added to water to form a syrup for fluid unit dosages. Hydro-alcoholic pharmaceutical preparations may be used having an acceptable sweetener, such as sugar, saccharin or a biological sweetener and a flavoring agent in the form of an elixir.

Pharmaceutical compositions for parenteral and suppository administration can also be obtained using techniques standard in the art.

A preferred use of the compounds according to the invention is as pharmaceutical agents suitable for oral administration. Another preferred use of the compounds is in transdermal parenteral cholinergic agonist and blocking pharmaceutical preparations, which are particularly useful in treating cholinergic disorders such as glaucoma, Myasthenia Gravis, Alzheimer's disease, and organophosphate poisoning. Accordingly, compositions suitable for administration to these areas are particularly included within the invention. The above parenteral solutions or suspensions may be administered transdermally and, if desired, a more concentrated slow release form may be administered. The above parenteral solutions or suspensions may be delivered with a skin patch. If desired these solutions or suspensions may be given by injection in an appropriate vehicle such as sesame oil.

Accordingly, incorporation of the active compounds and a slow release matrix may be implemented for administering transdermally. The compounds may be administered transdermally at about .01 to 99% of the composition and preferably about 25 to 85 wt% of the active ingredient in the vehicle or carrier.

Transdermal therapeutic systems are self-contained dosage forms that, when applied to intact skin, deliver drug(s) at a controlled rate to the systemic circulation. Advantages of using the transdermal routing include: enhanced therapeutic efficacy, reduction in the frequency of dosing, reduction of side effects due to optimization of blood-concentration vs. time profile, increased patient compliance due to elimination of multiple dosing schedules, bypassing the hepatic "first pass" metabolism, avoiding gastrointestinal incompatibilities and providing a predictable and extendable duration of activity. However, the main function of the skin is to act as a barrier to entering compounds. As a consequence, transdermal therapy has been preferred for a limited number of drugs that possess the desirable physiochemical properties for diffusion across the skin barrier. One effective method of overcoming the barrier function of the skin is to include a penetration enhancer in the formulation of the transdermal therapeutic system.

The penetration enhancer is a chemical compound that, when included in a formulation, temporarily increases the permeability of the skin to a drug line allowing more of the drug to be absorbed in a shorter period of time. Several different types of penetration enhancers have been reported such as dimethylsulfoxide, n-decylmethylsulfoxide, N,N-dimethylacetamide N,N-dimethylformamide, 1-dodecylazacycloheptane-2-one (Azone), propylene glycol, ethanol, pyrrolidones such as N-methyl-2-pyrrolidone (NMP) and surfactants.

The above compounds can be present in the reservoir alone or in combination with pharmaceutical carriers. The pharmaceutical carriers acceptable for the purposes of this invention are the art known carriers that do not adversely effect the drug, the host, or the material comprising the drug delivery device. Suitable pharmaceutical carriers include sterile water; saline; dextrose; dextrose in water or saline; condensation products of castor oil and ethylene oxide combining about 30 to 35 moles of ethylene oxide per mole of castor oil; liquid acid; lower alkanols; oils such as corn oil; peanut oil; sesame oil and the like, with emulsifiers such as mono- or di-glyceride of a fatty acid; or a phosphatide, e.g., lecithin, and the like; glycols; polyalkylene glycols; aqueous media in the presence of a suspending agent, for example, sodium carboxymethyl cellulose; sodium alginate; poly(vinylpyrrolidone); and the like, alone, or with suitable dispensing agents such as lecithin; polyoxyethylene stearate; and the like. The carrier may also contain adjuvants such as preserving, stabilizing, wetting, emulsifying agents and the like together with the penetration enhancer and the compounds of this invention.

The effective dose for mammals may vary due to such factors as age, weight, activity level or condition of the subject being treated. Typically, an effective dosage of a compound according to the present invention is about 1 to 800 milligrams when administered by either oral or rectal dose from 1 to 3 times daily. This is about .002 2 to about 50 milligrams per kilogram of the subject's weight administered per day. Preferably about 10 to about 300 milligrams are administered orally or rectally 1 to 3 times a day for an adult human. The required dose is considerably less when administered parenterally, preferably about .01 to about 150 milligrams may be administered intramuscularly or transdermally, one or two times a day for an adult human.

Compounds of the present invention may be administered topically at about .01 to about 99 wt% of the composition, and preferably about 25 to 85 wt%.

The present invention also provides compounds as defined above for inhibiting acetylcholinesterase activity, for the treatment of cholinergic disorders such as glaucoma, Myasthenia Gravis, Alzheimer's disease, or as an antidote for treating organophosphate poisoning in a mammal.

The invention further provides the use of a compound or composition as defined above in the preparation of a medicament for treating cholinergic disorders, such as glaucoma, Myasthenia Gravis and Alzheimer's disease, or for the treatment of organophosphate poisoning in a mammal.

Surprisingly, the compounds according to the invention have shown selective cholinergic agonist and blocking activity. Of the two enzymes known to hydrolyze acetylcholine in vivo, acetylcholinesterase (AChE) which is found in red blood cells, in the brain, and in nerve tissues, seems to be more specific then butyrylcholinesterase (BChE) which is found in serum, pancreas and in the liver. It, however, was never shown that compounds which selectively inhibit one of the two enzymes more than the other, would offer a medical advantage.

The present invention relates to selective inhibition as follows. The natural alkaloid (-)-physostigmine, its potential metabolite (-)-(N1)-norphysostigmine and the natural alkaloid physovenine which were used as biological standards in the inhibited AChE and BChE in vitro similarly at similar concentrations.

These biological standard compounds used for comparative purposes and derivatives having protective groups have the following structures. 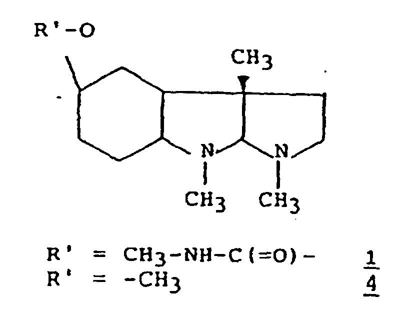 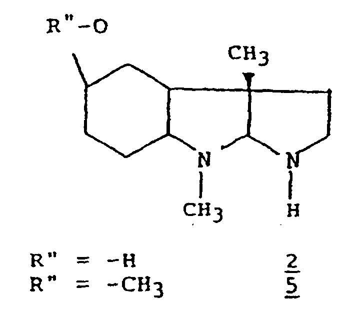 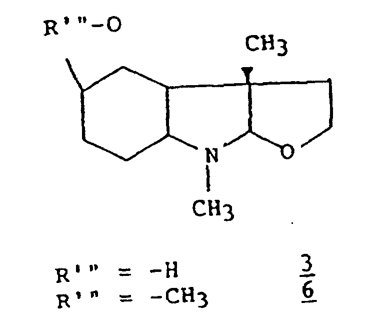 The above structures are also used as starting materials to produce compounds according to the present invention.

The phenylcarbamate of (-)-eseroline and referred to in the literature as phenserine, however, was determined by the present invention experimentation to inhibit AChE from human erythrocytes in vitro at a 50-times lower concentration than BChE from human plasma. Accordingly, further derivatives were made and tested.

It was discovered according to the present invention that substituting the phenyl group in para-position with a methyl group, a chlorine atom, or a methoxy group afforded derivatives which inhibited both enzymes at similar concentrations but such derivatives were considerably less potent than the biological standards described above. The phenylcarbamate of (-)-physovenol (22), also showed high preference for AChE (IC₅₀ for AChE = 11, and for RChE = 700), whereas the cumylcarbamate (4'-isopropylphenylcarbamate) (24) showed a reverse enzyme specificity (AChE = 3800 and for BChE = 16.5).

These above discoveries clearly indicated that selective inhibition of either AChE or BChE could be achieved by replacing the hydrogens on the phenyl group in phenylcarbamates with various substituents and inserting these modified phenylcarbamates on the basic core structure present in the three alkaloids that are the biological standards described above. The increased possibility of designing specifically acting inhibitors of AChE or BChE prompted an extension of these investigations and the results are the subject of the present invention.

The phenylcarbamates listed below in Table 1 and Table 2 were prepared from (-)-eseroline (4), (-)-N1)-benzylnoreseroline (5) as the N-protected equivalent of (2), and from (-)-physovenol (6) which all have the natural (3aS)-absolute configuration (these numbers for the starting materials correspond to the numbers on the comparative structures and protected derivatives, whose structures are listed just previously in the above specification).

Reaction of phenols having the natural (3aS)-absolute configuration, i.e., (-)-eseroline, (-)-(N1)-noreseroline, or (-)-(N1)-benzylnoreseroline with commercially available isocyanates in dry ether and in the presence of a catalytic amount of sodium, afforded the desired carbamates. See Reaction Scheme 1, below. They were separated from "dimers", which invariably formed, by chromatography, and removed as the faster running materials. The structures of the carbamates which often were amorphous were secured by MS and ¹H-NMR spectra, and they were characterized by TLC-analysis and by optical rotation. Details of the preparation of the carbamates according to the present invention are given in the experimental section. Conversion of the (N1)-protected carbamates into compounds of the (N1)-series was accomplished by catalytic hydrogenation over Pd(OH)₂ catalyst as shown in Scheme 2 and described below.

The resulting phenylcarbamates are listed below in Tables 1-2 , following the illustration of reaction Schemes 1 and 2. The phenyl carbamates, which all have the natural (3aS)-absolute-configuration, are listed below in Table 2.

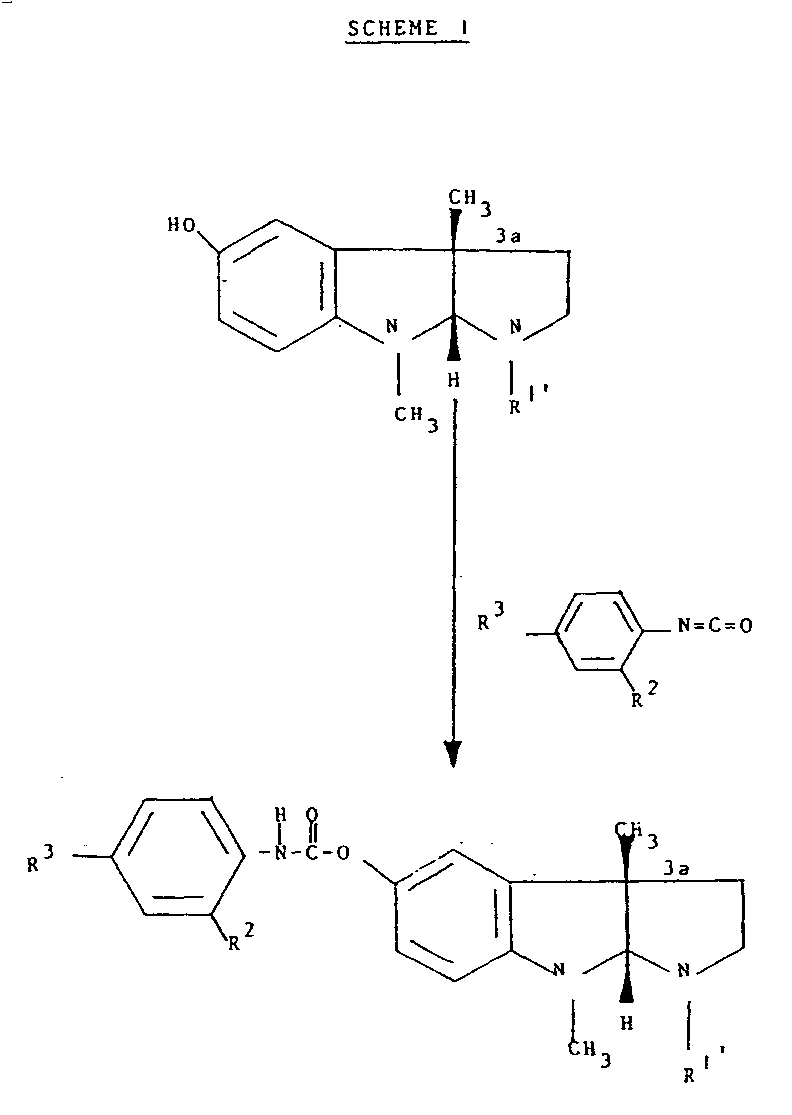

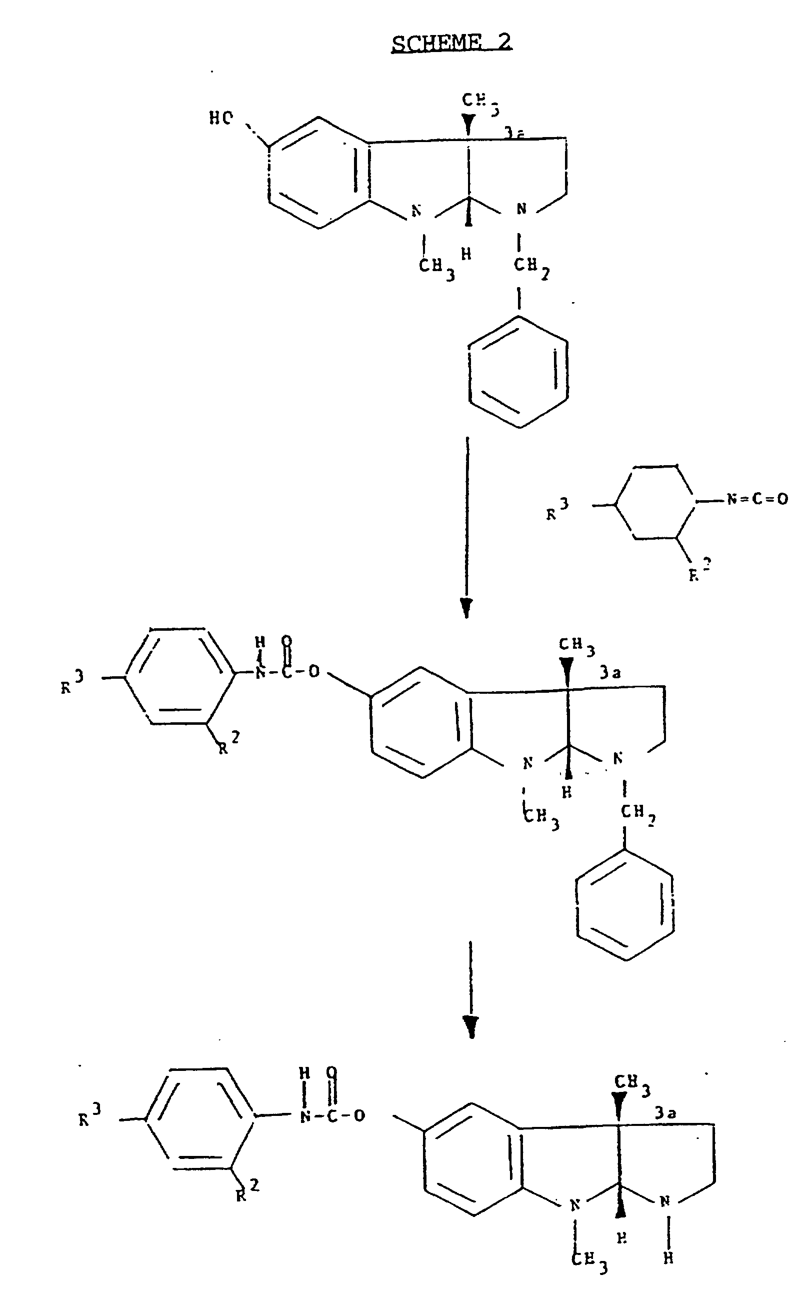

Compounds according to the present invention, i.e, compounds 7-12, are listed in Table 1 below.

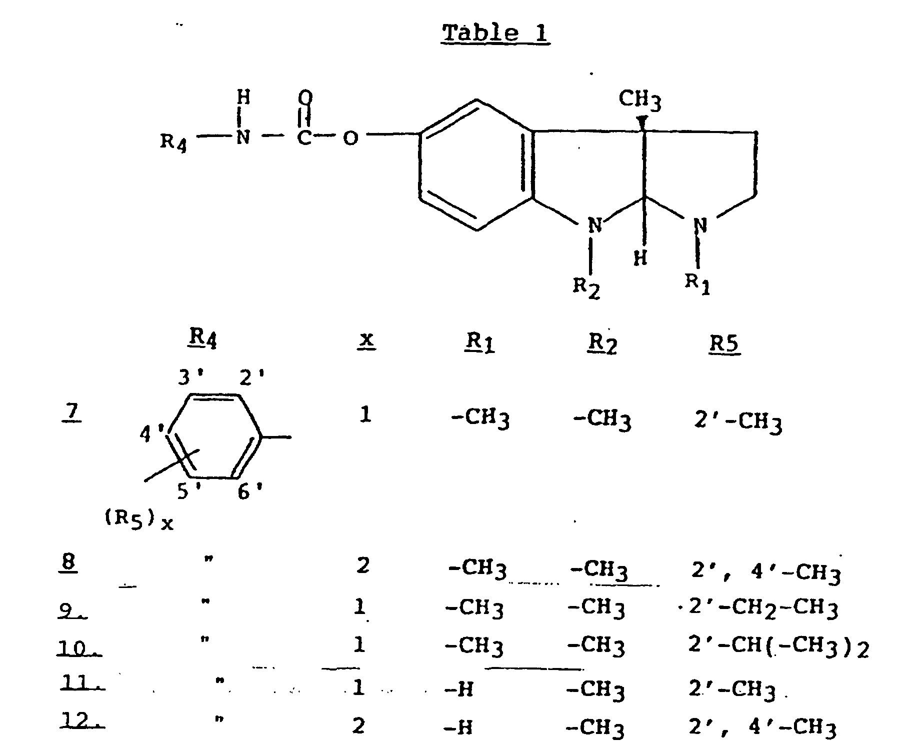

Compounds according to the present invention, i.e. compounds 13-19 and comparative compounds A', B', and C' are listed in Table 2, below.

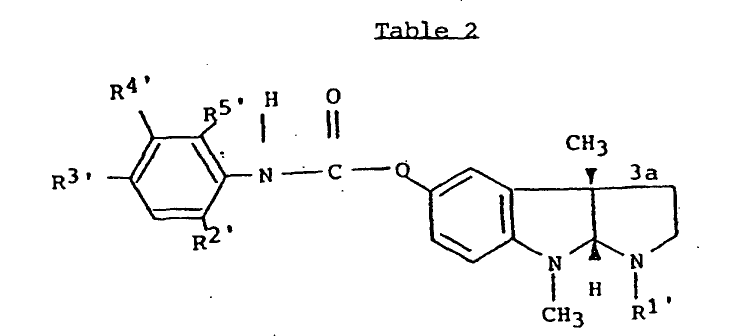

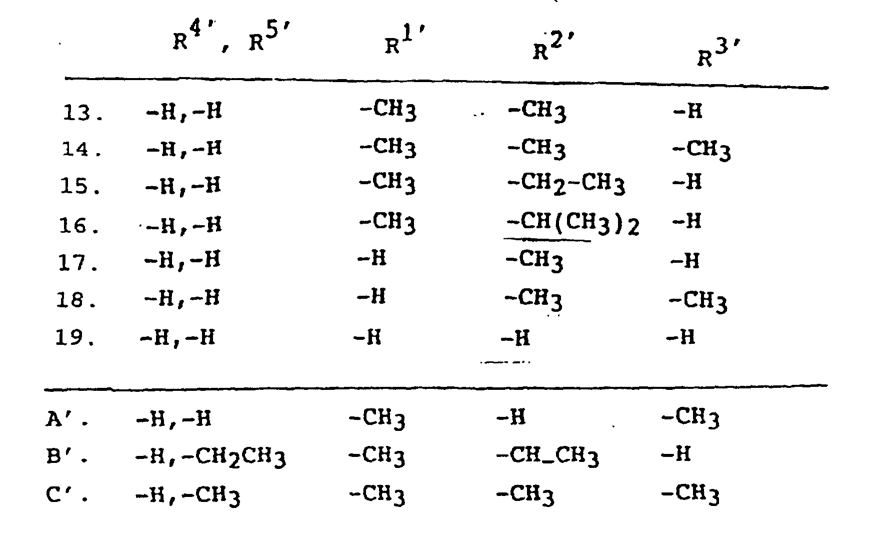

### Experimental

Melting points (uncorrected): Fisher-Johns apparatus; optical rotations ([α]_{D}, CHCl₃: Perkin-Elmer-241 MC automatic polarimeter, IR spectra (cm⁻¹, CHCl₃): Beckman-IR-4230 instrument, BIO-RAD FTS-45 instrument; ¹H NMR (in CDCl₃ with Me₄Si as internal reference, δ ppm, J Hz): Varian XL-300 MHz, Gemini 300 MH_{z} spectrometer, MS (m/z) for chemical ionization (CI): Finnigan-1015D mass spectrometer, for electron impact (EI): V.G. Micromass 7070 mass spectrometer, for HR MS (FAB): JEOL JMS-SX 102 magnetic sector mass spectrometer thin layer chromatography (silica gel GHLF), 250 µm): Analtech Inc.; column chromatography (silica gel GHLF, 250 µm); Merck 60 (230-400 mesh); the solvent systems used for TLC analysis were the following: CH₂Cl₂ /5% MeOH; CH₂Cl₂/10% MeOH; the solvent systems used for column chromatography: CH₂Cl₂/5% MeOH(A); CH₂Cl₂/10% MeOH(B).

### (-) -2'-Methylphenylcarbamoyleseroline (7):

(-)-Eseroline (4) 0 (0.12 g, 0.55 mmol) was dissolved in anhydrous Et₂O (10 mL) and a small piece of Na metal was added. After stirring for about 2 min at room temperature under nitrogen, 2-methylphenylisocyanate (0.09 g, 0.70 mmol) was added dropwise. After complete addition the solvent was evaporated immediately. The residue was flash chromatographed on a silica gel column (system B) to give (7) as a foam (0.88g, 46%); [α]_{D} -69 6° (c=0.5, CHCl₃), CI MS (m/z): 352 (M+⁺1); EI MS (m/z): 351 (M⁺), HR MS (FAB) calcd for (M⁺+1) C₂₁H₂₆N₃O₂ 352.2025, found 352.2020, IR; 3410, 2930, 1745; H NMR 1.46 (s, 3H, C10-CH₃), 1.90-2.12 (m 2H, c3-H), 2.32 (s, 3H, Me-ph), 2.55 (s, 3H, N1-CH₃, 2.58-2.70 (m, 2H, C2-H₂), 2.91 (s, 3H, N*-CH₃), 4.18 (s, 1H, C9-H), 6.33 (d, J = 8.4, 1H, C7-H), 6.63 (br s, 1H, N-H), 6.85-6.95 (m, 2H, C4-H, C6-H), 7.05 (t, J = 1H, C5'-H), 7.15-7.23 (m, CH, CH3'-H, C6'-H), 7.85 (br s, 1H, C4'-H). All other carbamates: (-)-2'-4'-dimethylphenylcarbamoyleseroline (8), (-)-2'-ethylphenylcarbamoyleseroline (9) and (-)-2'-isopropylphenylcarbamoyleseroline (10), were similarly prepared from (-)-eseroline (4) with the corresponding isocyanates and showed similar IR and NMR spectra to (7). The important data for these compounds is shown in Table 3 below.

The carbamates: (-)-2'methylphenylcarbamoyl-N1-noreseroline (11) and (-)-2"-4'-dimethylphenylcarbamoyl-N1-norseroline (12) were similarly prepared from (-)-(N1)-benzylnoreseroline (5) instead of (4) by reacting (5) with the corresponding isocyanates. The benzyl protecting group was then removed to yield the noreseroline compound from the benzylnoreseroline compound. The important data for these compounds is shown in Table 3 below.

The following example shows the removal of a benzyl protecting group from (-)-2'-Methylphenylcarbamoyl-(N1 )-benzylinoreseroline to yield compound (11).

### (-)-2' Methylphenylcarbamoyl-N1-Noreseroline (11) :

(-)-2'-Methylphenylcarbamoyl-(N1)-benzylinoreseroline (0.09g, 0.21 mmol) was dissolved in MeOH (10 mL), and palladium hydroxide on carbon (7 mg) was added. After hydrogenation under atmospheric pressure for 5 h, the palladium catalyst was filtered through Celite and the solvent was evaporated in vacuo. The residue was chromatographed by preparative TLC (silica gel plate 2000 µm, CH₂Cl₂/10% MeOH) to give compound (19) as a foam (0.04g, 56%): [α]_{D}-62.4° (c = 0.5, CHCl₃), CI MS (m/z):338 (M⁺+1); EI MS (m/z): 337 (M⁺), HR MS (EI) (M⁺) calcd, for C₂₀H₂₃N₃O₂ 337.1790, found 337.1776 ¹H NMR: 1.42 (s, 3H, C10-CH₃, 1.70-1.80 (m, 1H, C3-H), 1.95-2.08 (m, 1H, C2-H), 2.29 (s, 3H, C2'-CH₃), 2.70-2.80 (m, 1H, C2-H), 2.81 (s, 1H, N8-CH₃), 3.01-3.10 (ddd, J = 2.5, 2.5, 2.5, 1H, C2-H), 4.51 (s, 1H, C9-H), 6.25 (d, J = 9.0, 1H, C7-H), 6.63 (br s, 1H, N-H), 6.83-6.86 (m, 2H, C4-H, C6-H), 7.02 (t, J - 7.5, 1H, C5'-H), 7.15-7.22 (m, 2H, C3'-H, C6'-H), 7.85 (br s, 1H, C4'-H).

Compound (12) showed similar IR and NMR spectra to compound (11), see Table III below.

Table 3 below lists the important physical data for compounds according to the invention. The compound numbers in Table 3 correspond to the compound numbers in Table 1.

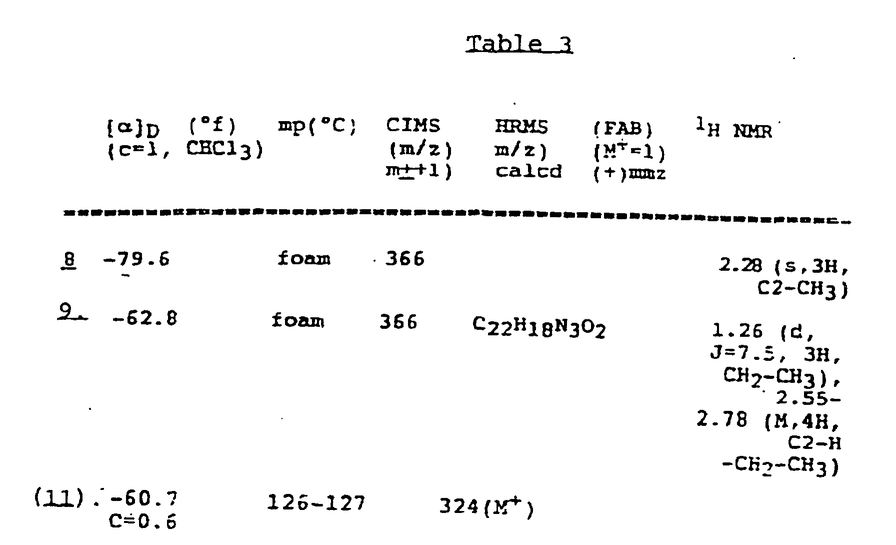

### EXAMPLE 13: (-)-2' -Methylphenylcarbamoyleseroline

(-)-Eseroline 0 (0.12 g, 0.55 mmol) was dissolved in anhydrous Et₂O (10 mL) and a small piece of Na metal was added. After stirring for about 2 min at room temperature under nitrogen, 2'-methylphenylisocyanate (0.09 g, 0.70 mmol) was added dropwise. After complete addition the solvent was evaporated immediately. The residue was flash chromatographed on a silica gel column (system B) to give (-)-2'-methylphenylcarbamoyleseroline as a foam (0.88g, 46%); [α]_{D}-69.6° (c=0.5, CHCl₃), CI MS (m/z): 352 (M⁺+1); EI MS (m/z): 351 (M⁺), HR MS (FAB) calcd for (M⁺+1) C₂₁H₂₆N₃O₂ 352.2025, found 352.2020, IR; 3410, 2930, 1745; ¹H NMR 1.46 (s, 3H, C10-CH₃), 1.90-2.12 (m 2H, c3-H), 2.32 (s, 3H, Me-Ph), 2.55 (s, 3H, N1-CH₃, 2.58-2.70 (m, 2H, C2-H₂), 2.91 (s, 3H, N*-CH₃), 4.18 (s, 1H, C9-H), 6.33 (d, J = B.4, 1H, C7-H), 6.63 (br s, 1H, N-H), 6.85-6.95 (m, 2H, C4-H_{,} C6-H), 7.05 (t, J = 1H, C5'-H), 7.15-7.23 (m, CH, CH3'-H, C6'-H), 7.85 (br s, 1H, C4'-H).

All other carbamates: (-)-2'-4'-dimethylphenylcarbamoyleseroline, 4'-isopropylcarbamoyleseroline, (-)-2'-ethylphenylcarbamoyleseroline, (-)-2'isopropylphenylcarbamoyleseroline, naphthylcarbamoyleseroline; were similarly prepared from (-)-eseroline with the corresponding isocyanates. The important physical data for these compounds are shown below.

### EXAMPLE 14

### (-)-2'-4'-Dimethylphenyl-carbamoyleseroline

The important data is as follows: a foam, [α]_{D}-79.6° (c=1, CHCl₃), CI MS (m/z): 366; ¹H NMR 2.28 (s, 3H, C2'-CH₃), 2.29 (s, 3H, C4'-CH₃₎.

### EXAMPLE 15

### (-)-2'-Ethylphenylcarbamoyleseroline

The important data is as follows: a foam, [a]_{D} -62.8° (c=1, CHCl₃) CI MS (m/z) 366 (M⁺= 1); HR MS (FAB): calcd for (M⁺+1) C₂₂H₂₈N₃O₂, 366.2182; ¹H-NMR: 1.26 (t, J=7.5, 3H, -CH₂-CH₃), 2.55-2.78 (m, 4H, C2-H, -CH₂-CH₃).

### EXAMPLE 16

### (-)-2'-Isopropylphenylcarbamoyleseroline

The important data is as follows: a foam, [a]_{D} -58.8° (c=1, CHCl₃) CI MS (m/z) 380 (M⁺= 1); HR MS (FAB): calcd for (M⁺+1) C₂₃H₂₉N₃O₂, 379.2259; ¹H-NMR: 1.30(d, J=6.8, 6H, -CH-(CH₃)₂).

### EXAMPLE 17

### (-) -2' -Methylphenylcarbamoyl-N1-noreseroline

### (a)(-)-2'-Methylphenylcarbamoyl-N1-benzylnoreseroline

(N¹ )-Benzylnoreseroline (2.0 g) was dissolved in anhydrous Et₂O (10 ml), and a small piece of Na added. After stirring for 2 minutes at room temperature under nitrogen, 2-methylphenyl isocyanate was added (0.04 g). After stirring for 15 minutes the solvent was evaporated and the residue was chromatographed to give the carbamate as a foam: [α]_{D} -62.0° (c=0.5, CHCl₃); CI MS (m/z) 428 (M⁺ + 1).

### (b) (-)-2'-Methylphenylcarbamoyl-N1-noreseroline

(-)-2'-Methylphenylcarbamoyl-N1-benzylnoreseroline (0.09g, 0.21 mmol) from (a) above was dissolved in MeOH (10 mL), and palladium hydroxide on carbon (7 mg) was added. After hydrogenation under atmospheric pressure for 5 h, the palladium catalyst was filtered through Celite and the solvent was evaporated in vacuo. The residue was chromatographed by preparative TLC (silica gel plate 2000 µm, CH₂Cl₂/10% MeOH) to give (-)-2'-methylphenylcarbamoyl-N1-noreseroline as a foam (0.04g, 56%): [α]_{D}-62.4° (c = 0.5, CHCl₃), CI MS (m/z):338 (M⁺+1); EI MS (m/z): 337 (M⁺), HR MS (EI) (M⁺) calcd, for C₂₀H₂₃N₃O₂ 337.1790, found 337.1776 ¹H NMR: 1.42 C20H23N302 337.1790, found 337.1776 ¹H NMR: 1:42 (s, 3H, C10-CH₃), 1.70-1.80 (m, 1H, C3-H), 1.95-2.08 (m, 1H, C2-H), 2.29 (s, 3H, C2'-CH₃), 2.70-2.80 (m, 1H, C2-H), 2.81 (s, 1H, N8-CH₃), 3.01-3.10 (ddd, J = 2.5, 2.5, 2.5, 1H, C2-H), 4.51 (s, 1H, C9-H), 6.25 (d, J = 9.0, 1H, C7-H), 6.63 (br s, 1H, N-H), 6.83-6.86 (m, 2H, C4-H, C6-H), 7.02 (t, J - 7.5, 1H, C5'-H), 7.15-7.22 (m, 2H, C3'-H, C6'-H), 7.85 (br s, 1H, C4'-H).

### EXAMPLES 18 and 19

The carbamates of Examples 18 and 19, belonging to the(-)-N¹-noreseroline series, were prepared from Examples belonging to the (-)-N¹-benzylnoreseroline series, by catalytic debenzylation as described in Example 17. The important physical data is as follows.

### EXAMPLE 18

### (-)-2'-4'-Dimethylphenylcarbamoyl-(N1)-noreseroline

The important data is as follows: a foam, [a]_{D} -55.4° (C=0.5 CHCl₃), CI MS (m/z): 352; ¹H NMR 2.45 (2s, 6H).

### EXAMPLE 19

### (-)-Phenylcarbamoyl-(N1)-noreseroline

The important data is as follows: m.p. (°C) 129-131, [α]_{D} -50.4° (c=0.5, CHCl₃) CI MS (m/z) 324 (M⁺+1); ¹H-NMR: 7.25-7.52 (m, 5H).

The following inactive compounds were provided using the above method.

### EXAMPLE A'

### (-)-4'-methylphenylcarbamoyleseroline

The important data is as follows: m.p. (°C) 143-145 [α]_{D} -74.2° (c=1, CHCl₃) CI MS (m/z) 352 (M⁺= 1); HR MS (FAB): calcd for (M⁺ +1) C₂₁H₂₆N₃O₂, _{352.2025} 1_{H-} NMR:2.31 (s,3H, C4'-CH₃).

### EXAMPLE B'

### (-)-2',6'-diethylphenylcarbamoyleseroline

The important data is as follows: an oil, [α]_{D} -36.1° (c=1, CHCl₃) CI MS (m/z) 394 (M⁺= 1); HR MS (FAB): calcd for (M⁺+1) C₂₄H₃₂N₃O₂, 394.2495; 1_{H-NMR:} 1.24 (t, J=7.4, 6H, 2-CH₂-CH₃), 2.68 (m, 6H, C2-H, 2-CH₂CH₃₎.

### EXAMPLE C'

### (-)-2',4',6'-trimethylphenylcarbamoyleseroline

The important data is as follows: a foam, [α]_{D} -55.8° (c=1, CHCl₃) CI MS (m/z) 380 (M⁺= 1); ¹H-NMR: 2.28(3s, 9H, C2', C4', C6'-CH₃).

The comparative (-)-phenylcarbamoyleseroline compound ((-)-phenserine) was provided as follows.

### EXAMPLE D': (-)-phenylcarbamoyleseroline:

(-)-Eseroline 0 (0.12 g, 0.55 mmol) was dissolved in anhydrous Et₂O (10 mL) and a small piece of Na metal was added. After stirring for about 2 min at room temperature under nitrogen, phenylisocyanate (0.09 g, 0.70 mmol) was added dropwise. After complete addition the solvent was evaporated immediately. The residue was flash chromatographed on a silica gel column (system B) to give (-)-phenylcarbamoyleseroline mp(°C) 142-143 (0.88g, 46%); [α]_{D}-74.2° (c=1, CHCl₃), CI MS (m/z): 338; 1_{H} NMR 7.01 (t, J=7.4, 1H, C4'-H), 7.22 (t, J7.4, 2H, C3'-H, C5'-H), 7.34 (d, J=7.4, 2H, C2'-H, C6' -H). The common name for this compound is (-)-phenserine.

The compound numbers in Tables 2 and 4 correspond to one another and to the above Examples. Comparative Example D' is (-)-Phenserine.

Table 4 below lists the important physical data for compounds according to the invention, which correspond to the compound numbers in Table 2.

**Table 4**

| IC₅₀ Values of Phenylcarbamates of (-)-Eseroline, (-)-Physovenol, and (-)- N¹-Noreseroline vs. Human Erythrocyte AChE and Human Plasma BChE | | |
|---|---|---|
| | IC₅₀ [nmol] | |
| No. | AChE | BChE |
| Biological standards | | |
| A physostignine | 27.9 ± 2.4 | 16.0 ± 2.9 |
| B N' norphysostigmine | 21.0 ± 1.0 | 2.0 ± 1.0 |
| C physovenine | 27.1 ± 0.8 | 3.7 ± 1.4 |
| D' phenserine | 24.0 ± 6.0 | 1300 ± 85.0 |

| Examples | | |
|---|---|---|
| Ex. 13 | 10.3 ± 1.6 | 1948.5 ± 245.5 |
| Ex. 14 | 13.6 ± 1.0 | 1817.0 ± 558.5 |
| Ex. 15 | 9.7 ± 0.7 | 2916.0 ± 537.0 |
| Ex. 16 | 15.5 ± 1.3 | 647.8 ± 46.2 |
| Ex. 17 | 17.0 ± 0.2 | 2165.0 ± 85.0 |
| Ex. 18 | 17.3 ± 1.2 | 1139.0 ± 26.0 |
| Ex. 19 | 13.8 ± 0.7 | 612.0 ± 0.4 |

| Inactive Compounds | | |
|---|---|---|
| Ex. A' | 139.2 ± 3.7 | 251.1 ± 8.6 |
| Ex. B' | 1493.7 ± 49.8 | 1073.5 ± 48.0 |
| Ex. C' | 1291.9 ± 73.8 | 1817.0 ± 885.0 |

In vitro assay of human anti-AChE and -BChE activity, IC₅₀

A classical enzyme inhibition assay was undertaken to quantitate the activity of the derivatives against AChE and BChE. Anti-cholinesterase activity was determined against human erythrocyte AChE and plasma BChE in 0.1 M Na₃PO₄ buffer (pH 8.0) using the spectrophotometric method of Ellman et al. (Biochem. Pharmacol. 7:88, 1961). Freshly collected plasma was diluted 1.125 with 0.1 M Na₃PO₄ (pH 7.4) and lysed erythrocytes similarly diluted to 1:200. Acetyl-B-methylthiocholine (0.5 mM) and s-butyrylthiocholine (0.5 mM) were used as specific substrates for AChE and BChE, respectively, 25 µl of substrate and 25 µl of enzyme in a total volume of 0.75 ml.

Physostigmine derivatives, diluted in half log-intervals to a concentration range of between 1x10⁻⁵M and 3x10⁻¹⁰M, were preincubated with enzyme (30 min at 21°C) prior to addition of substrates. Following incubation (30 min at 37°C), production of a yellow thionitrobenzoate anion was measured with a spectrophotometer set to 412 nm wavelength. Nonspecific substrate hydrolysis was determined under conditions of complete enzyme inhibition (by addition of physostigmine 1x10⁻⁵ M), and the associated change in absorbance subtracted from that observed with the test compounds. Finally, the activity of each compound was assessed alongside that of physostigmine, as an external standard, whose activity has been previously reported (Atack et al., J. Pharm. Expl. Ther. 249:294, 1989).

The AChE and BChE activity of each compound was expressed as an IC₅₀, which is defined as the concentration in nmol required to inhibit 50% of enzyme activity (calculated as described by Atack et al., J. Pharm. Expl. Ther. 249:294, 1989)).

### In vivo duration of activity studies

Catheters, filled with heparinized saline, were tied into the right femoral vein and artery of anesthetized male rats, which then were restrained in a plaster cast and allowed to recover from anesthesia in a temperature-controlled enclosure. Plasma samples were withdrawn to quantitrate untreated levels of AChE activity. At 90 min. after surgery, hexamethonium bromide (5 mg/kg, i.p.) was administered, followed by atropine methylbromide (4 mg/kg, s.c.) 10 min. later. These quaternary nicotinic and muscarinic antagonists, do not enter brain and inhibit peripheral cholinergic overdrive associated with cholinesterase inhibition, which may be deleterious to the animal. Two hours after surgery, either (i) physostigmine, (ii). physostigmine. derivatives, or (III) THA was administered i.v. Plasma samples were removed at intervals between 2 min. and 8 hr., immediately frozen to -70°C and then assayed for cholinesterase inhibition. AChE inhibition was measured as described above, with necessary modifications required for quantitation from rat plasma.

All drugs were formulated in a manner consistent with i.v. administration. Specifically, drugs were dissolved in Tween 80/EtOH (3:1, V:V), approximately 100 µl, and then were diluted in excess of 1:9 (V:V) with isotonic saline. The use of Tween 80/EtOH did not affect either AChE or BChE inhibitory activity of compounds in in vitro studies (Yu et al., Helv. Chim. Acta 74, pages. 761-766, (1991)). Doses were determined in prior studies involving the measurement of rectal temperature and tremor; two centrally-mediated actions of cholinesterase inhibitiors and cholinergic agonists.

Figure 1 demonstrates the in vivo inhibition of the enzyme acetylcholinesterase (AChE) by physostigmine and its 2',4'-dimethylphenyl carbamate derivative, i.e., the time-dependent activity of these cholinesterase inhibitors in rats. As predicted from the in vitro IC₅₀ studies, physostigmine and the substituted phenyl carbamates to which this patent relates (which are represented in this case by 2',4'-dimethylphenyl physostigmine) possess excellent in vivo cholinesterase inhibitory properties. However, the duration of enzyme inhibition is short following an intravenous bolus of physostigmine. Whereas as peak inhibition of 46% occurred within 2 minutes of administration, this rapidly declined to 25% by 15 minutes and was negligible at one hour. An equal dose of the 2' 4 '-methylphenyl carbamate resulted in immediate 60% AChE inhibiiton at 2 minutes. This was maintained at a steady level for 2 hours and then slowly declined to 36% inhibition at 8 hours. The high activity, specificity and persistence of 2',4'-dimethylphenyl physostigmine, which is achieved without side-effects or toxicity, is surprising and supports the contention that these compounds represent a class of potent, new and selective cholinesterase inhibitors.

## Claims

1. A compound according to the formula: 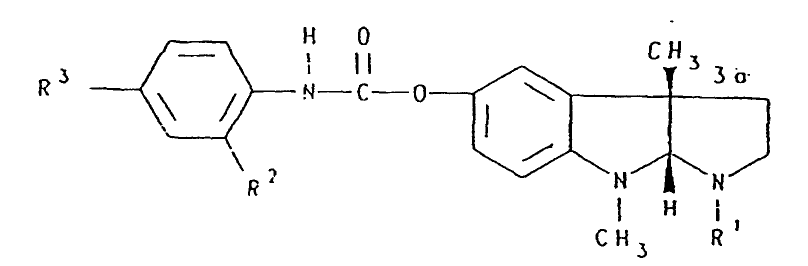 wherein R¹ is H or a -CH₃ group,
R² is a straight or branched chained C₁-C₃ alkyl,
R³ is H or a straight or branched C₁-C₃ alkyl,
and pharmaceutically acceptable salts.

2. A compound as claimed in Claim 1 in which R¹ is hydrogen.

3. A compound as claimed in Claim 1 in which R¹ is -CH₃.

4. A compound selected from:
(-)-2'-methylphenylcarbamoyleseroline;
(-)-2', 4'-dimethylphenylcarbamoyleseroline;
(-)-2' -ethylphenylcarbamoyleseroline;
(-)-2'-isopropylphenylcarbamoyleseroline;
(-)-2'-methylphenylcarbamoyl-Nl-noreseroline;
(-)-2', 4'-dimethylphenylcarbamoyl-N1-noreseroline;
(-)-phenylcarbamoyl-N1-noreseroline;
and pharmaceutically acceptable salts thereof.

5. (-)-2'-Methylphenylcarbamoyleseroline or a pharmaceutically acceptable salt thereof.

6. (-)-2', 4'- Dimethylphenylcarbamoyleseroline or a pharmaceutically acceptable salt thereof.

7. (-)-2'-Ethylphenylcarbamoyleseroline or a pharmaceutically acceptable salt thereof.

8. (-)-2'-Isopropylphenylcarbamoyleseroline or a pharmaceutically acceptable salt thereof.

9. (-)-2'Methylphenylcarbamoyl-N1-noreseroline or a pharmaceutically acceptable salt thereof.

10. (-)-2',4'-Dimethylphenylcarbamoyl-N1-noreseroline or a pharmaceutically acceptable salt thereof.

11. (-)-Phenylcarbamoyl-N1-noreseroline or a pharmaceutically acceptable salt thereof.

12. A pharmaceutical composition comprising a pharmaceutically effective amount of a compound according to any of claims 1 to 11 and a carrier.

13. A compound as claimed in any of claims 1 to 11 or a composition as claimed in Claim 12 for use in the treatment of cholinergic disorders.

14. A compound as claimed in any of claims 1 to 11 or a composition as claimed in Claim 12 for use in the treatment of glaucoma, Myasthenia Gravis or Alzheimer's disease.

15. A compound as claimed in any of claims 1 to 11 or a composition as claimed in Claim 12 for inhibiting acetylcholinesterase activity.

16. A compound as claimed in any of claims 1 to 11 or a composition as claimed in Claim 12 for treating organophosphate poisoning in a mammal.

17. The use of a compound as claimed in any of claims 1 to 11 or a composition as claimed in Claim 12 in the preparation of a medicament for treating cholinergic disorders.

18. The use as claimed in Claim 17 in which the compound is to be administered transdermally.

19. The use of a compound as claimed in any of claims 1 to 11 or a composition as claimed in Claim 12 in the preparation of a medicament for the treatment of glaucoma, Myasthenia Gravis or Alzheimer's disease.

20. The use of a compound as claimed in any of claims 1 to 11 or a composition as claimed in Claim 12 in the preparation of a medicament for the treatment of organophosphate poisoning in a mammal.

## Patentansprüche

1. Verbindung entsprechend der Formel: 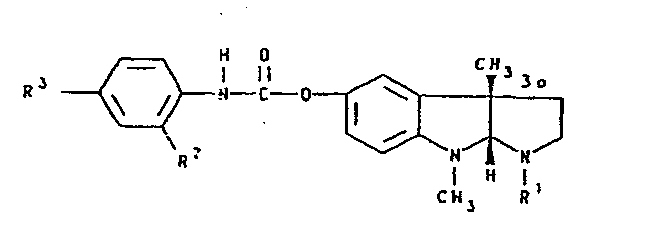 worin R¹ H oder eine -CH₃-Gruppe darstellt,
R² ein geradkettiges oder verzweigtes C₁-C₃-Alkyl darstellt,
R³ H oder ein geradkettiges oder verzweigtes C₁-C₃-Alkyl darstellt und pharmazeutisch akzeptable Salze.

2. Verbindung nach Anspruch 1, worin R¹ Wasserstoff darstellt.

3. Verbindung nach Anspruch 1, worin R¹ -CH₃ darstellt.

4. Verbindung ausgewählt aus:
(-)-2'-Methylphenylcarbamoyleserolin;
(-)-2',4'-Dimethylphenylcarbamoyleserolin;
(-)-2'-Ethylphenylcarbamoyleserolin;
(-)-2'-Isopropylphenylcarbamoyleserolin;
(-)-2'-Methylphenylcarbamoyl-N1-noreserolin;
(-)-2',4'-Dimethylphenylcarbamoyl-N1-noreserolin;
(-)-Phenylcarbamoyl-N -noreserolin
und pharmazeutisch akzeptable Salze hiervon.

5. (-)-2'-Methylphenylcarbamoyleserolin oder ein pharmazeutisch akzeptables Salz hiervon.

6. (-)-2',4'-Dimethylphenylcarbamoyleserolin oder ein pharmazeutisch akzeptables Salz hiervon.

7. (-)-2'-Ethylphenylcarbamoyleserolin oder ein pharmazeutisch akzeptables Salz hiervon.

8. (-)-2'-Isopropylphenylcarbamoyleserolin oder ein pharmazeutisch akzeptables Salz hiervon.

9. (-)-2'-Methylphenylcarbamoyl-N1-noreserolin oder ein pharmazeutisch akzeptables Salz hiervon.

10. (-)-2',4'-Dimethylphenylcarbamoyl-N1-noreserolin oder ein pharmazeutisch akzeptables Salz hiervon.

11. (-)-Phenylcarbamoyl-N1-noreserolin oder ein pharmazeutisch akzeptables Salz hiervon.

12. Pharmazeutische Zusammensetzung, umfassend eine pharmazeutisch wirksame Menge einer Verbindung nach irgendeinem der Ansprüche 1 bis 11 und einen Träger.

13. Verbindung nach irgendeinem der Ansprüche 1 bis 11 oder Zusammensetzung nach Anspruch 12 zur Verwendung bei der Behandlung von cholinergen Störungen.

14. Verbindung nach irgendeinem der Ansprüche 1 bis 11 oder Zusammensetzung nach Anspruch 12 zur Verwendung bei der Behandlung von Glaucomen, Myasthenia Gravis oder der Alzheimer-Erkrankung.

15. Verbindung nach irgendeinem der Ansprüche 1 bis 11 oder Zusammensetzung nach Anspruch 12 zur Inhibierung der Acetylchotinesteraseaktivität.

16. Verbindung nach irgendeinem der Ansprüche 1 bis 11 oder Zusammensetzung nach Anspruch 12 zur Behandlung einer Organophosphatvergiftung in einem Säugetier.

17. Verwendung einer Verbindung nach irgendeinem der Ansprüche 1 bis 11 oder einer Zusammensetzung nach Anspruch 12 zur Herstellung eines Arzneimittels für die Behandlung cholinerger Störungen.

18. Verwendung nach Anspruch 17, worin die Verbindung transdermal verabreicht wird.

19. Verwendung einer Verbindung nach irgendeinem der Ansprüche 1 bis 11 oder einer Zusammensetzung nach Anspruch 12 zur Herstellung eines Arzneimittels für die Behandlung von Glaucomen, Myasthenia Gravis oder der Alzheimer-Erkrankung.

20. Verwendung einer Verbindung nach irgendeinem der Ansprüche 1 bis 11 oder einer Zusammensetzung nach Anspruch 12 zur Herstellung eines Arzneimittels für die Behandlung einer Organophosphatvergiftung in einem Säugetier.

## Revendications

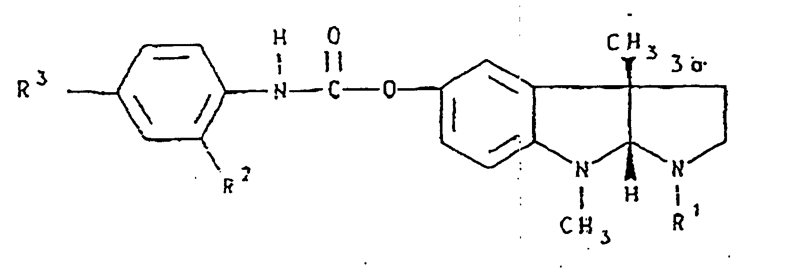

1. Composé selon la formule ci-dessus, dans laquelle R¹ représente H ou un groupe -CH3,
R² est une chaîne alkyle C₁-C₃ ramifiée ou droite
R³ représente H ou un alkyle C₁-C₃ droit ou ramifié, et sels pharmaceutiquement acceptables.

2. Composé selon la revendication 1 selon lequel R¹ est de l'hydrogène.

3. Composé selon la revendication 1 selon lequel R¹ représente -CH₃.

4. Composé sélectionné parmi :
(-)-2'-méthylphénylcarbamoyleséroline ;
(-)-2', 4'-diméthylphénylcarbamoyleseroline ;
(-)-2'-éthylphénylcarbamoyleseroline;
(-)-2'-isopropylphénylcarbamoyleseroline;
(-)-2'-méthylphénylcarbamoyl-N1-noreseroline;
(-)-2', 4'-dlméthylphény(carbamoyl-N1-noreseroline;
(-)-phénylcarbamoyl-N1-noreseroline;et sels pharmaceutiquement acceptables de ces composés.

5. (-)-2'- Méthylphénylcarbamoyleseroline ou sel pharmaceutiquement acceptable de ce composé.

6. (-)-2', 4'- Diméthylphénylcarbamoyleseroline ou sel pharmaceutiquement acceptable de ce composé.

7. (-)-2'- Ethylphénylcarbamoyleseroline ou sel pharmaceutiquement acceptable de ce composé.

8. (-)-2'- Isopropylphénylcarbamoyleseroline ou sel pharmaceutiquement acceptable de ce composé.

9. (-)-2'- méthylphénylcarbamoyl-N1-noreseroline ou sel pharmaceutiquement acceptable de ce composé.

10. (-)-2'-4'-Diméthylphénylcarbamoyl-N1-noreseroline ou sel pharmaceutiquement acceptable de ce composé.

11. (-)- Phénylcarbamoyl-N1-noreseroline ou sel pharmaceutiquement acceptable de ce composé.

12. Composition pharmaceutique comprenant une quantité pharmaceutiquement efficace d'un composé selon l'une quelconque des revendications 1 à 11 et un excipient.

13. Composé selon l'une quelconque des revendications 1 à 11, ou composition selon la revendication 12 prévue pour être utilisée dans le traitement des troubles cholinergiques.

14. Composé selon l'une quelconque des revendications 1 à 11 ou composition selon la revendication 12 prévue pour être utilisée dans le traitement du glaucome, de la myasthénie ou de la maladie d'Alzheimer.

15. Composé selon l'une quelconque des revendications 1 à 11, ou composition selon la revendication 12 permettant d'inhiber l'activité de l'acétylcholinestérase.

16. Composé selon l'une quelconque des revendications 1 à 11 ou composition selon la revendication 12 pour traiter l'empoisonnement à l'organophosphate chez un mammifère.

17. Utilisation du composé selon l'une quelconque des revendications 1 à 11 ou composition selon la revendication 12 pour la préparation d'un médicament permettant de traiter les troubles cholinergiques.

18. Utilisation selon la revendication 17, selon laquelle le composé peut être administré par voie transdermique.

19. Utilisation d'un composé selon l'une quelconque des revendications 1 à 11 ou composition selon la revendication 12 dans la préparation d'un médicament pour le traitement du glaucome, de la myasthénie ou de la maladie d'Alzheimer.

20. Utilisation d'un composé selon l'une quelconque des revendications 1 à 11, ou composition selon la revendication 12 pour la préparation d'un médicament pour le traitement de l'empoisonnement à l' organophosphate chez un mammifère.
